# EUROPÄISCHE PATENTSCHRIFT

(11) **EP 2 667 907 B1**
(45) Veröffentlichungstag und Bekanntmachung des Hinweises auf die Patenterteilung: **30.03.2016**
(21) Anmeldenummer: 12702424.8
(22) Anmeldetag: 24.01.2012
(51) Int. Cl.: A61M 1/14, A47F 7/14

(54) **HALTERUNG FÜR EINE GEBRAUCHSANWEISUNG**
HOLDER FOR AN INSTRUCTION MANUAL
SUPPORT POUR UN MODE D'EMPLOI

(30) Priorität: 25.01.2011 DE 102011009396
(43) Veröffentlichungstag der Anmeldung: 04.12.2013
(73) Patentinhaber: Fresenius Medical Care Deutschland GmbH, 61352 Bad Homburg (DE)
(72) Erfinder: FÖRGER, Jens, 35789 Laubuseschbach (DE); OESTERREICH, Stefan, 61267 Neu-Anspach (DE)
(74) Vertreter: Ziermann, Oliver
(86) Internationale Anmeldenummer: PCT/EP2012/000304
(87) Internationale Veröffentlichungsnummer: WO 2012/100935

(56) Entgegenhaltungen:
- WO-A1-2010/107455
- DE-U1- 20 202 774
- US-A- 3 322 381
- US-A- 5 215 379
- US-A1- 2007 299 358

## Beschreibung

Die Erfindung betrifft eine Halterung für die Anbringung einer Informationsanzeige, z.B. einer Gebrauchsanweisung, an einer Stange, z.B. der Infusionsstange, eines medizinischen Geräte nach dem Oberbegriff des Anspruchs 1. Weiterhin betrifft die Erfindung die Verwendung der Halterung nach Anspruch 14 zur Bereitstellung von Informationen für Pflege- und Servicepersonal an einem medizinischen Gerät.

Moderne medizinische Geräte zur Behandlung von Patienten, wie z.B. Dialysemaschinen oder Geräte für die Bluttransfusion, stellen bei Wartung und Bedienung häufig hohe Anforderungen an den Techniker oder das Pflegepersonal.

Sie sind meist in der Lage, unterschiedliche Behandlungsverfahren durchzuführen und bestehen aus einer Vielzahl unterschiedlicher Komponenten. Sowohl bei der Wartung und Vorbereitung als auch während der Behandlung in der Klinik stehen dem Bediener viele Optionen zur Verfügung, oft sind komplexe Arbeitsschritte zu befolgen. Die Anleitung dazu kann der Bediener z.B. in der Gebrauchsanweisung der Maschine finden. Es ist also vorteilhaft, wenn diese Gebrauchsanweisung während der Wartung oder Bedienung des Gerätes einfach einzusehen ist. Damit kann die Arbeitszeit effizienter genutzt werden, da dem Bediener die notwendigen Informationen jederzeit sofort zur Verfügung stehen. Aus hygienischen und organisatorischen Gründen ist es ungünstig, dass diese Gebrauchsanweisung auf der Maschine oder anderen Ablageflächen am Behandlungsplatz aufbewahrt wird. Eine Befestigung an einer geeigneten Stelle des medizinischen Gerätes, an der der normale Behandlungsablauf nicht gestört wird, ist zu bevorzugen.

So ist in der US 4,867,738 ein Autotransfusionssystem beschrieben, bei dem die Gebrauchsanweisung in Form einer Tafel fest über eine Klebeverbindung mit dem dort verwendeten Einwegartikel verbunden ist. So stehen dem Benutzer die Anweisungen direkt während der Anwendung der Vorrichtung zur Verfügung. Allerdings ist die Fläche für die Informationsdarstellung beschränkt. Durch die Klebeverbindung mit dem Disposable ist sie nur einmal verwendbar.

Darüberhinaus sind Vorrichtungen bekannt, die die Anbringung und Aufbewahrung unterschiedlichster Gegenstände direkt an einer Infusionsstange ermöglichen.

In der US 2007/0267551 ist eine Halterung für eine Infusionsstange beschrieben, die zur Aufnahme der persönlichen Gegenstände eines Patienten dient, damit er diese zusammen mit der Infusionsstange ständig mit sich führen kann. Diese Halterung wird mit einer Schraubklemme an der Infusionsstange befestigt. Solche Schraubklemmen bestehen aus mehreren Teilen und sind durch ihren Aufbau nur sehr schwer zu reinigen. Im medizinischen Umfeld spielt die Hygiene eine große Rolle und um eine einfache Reinigung und Desinfektion zu ermöglichen, werden glatte Flächen bevorzugt.

In der US 2005/0016044 erfolgt die Befestigung von Gegenständen, z.B. von Rahmen, an der Infusionsstange mithilfe einer biegsamen Metallkabels, das um die Stange gewickelt wird. Die Montage und auch die Deinstallation sind eher umständliche Arbeiten.

In der US 2009/0294604 sind Befestigungsmittel beschrieben, die mit einer Hand angebracht werden können, und deren Fixierung an der Stange über Reibung erfolgt. Ein Nachteil dieser Vorrichtung ist, dass sie ohne Belastung leicht verrutschen kann. Deshalb müssen in der Infusionsstange spezielle Klemmbacken vorgesehen sein, um die Halterungen in der gewünschen Position zu halten, wenn bei fehlender Gewichtsbelastung die Reibung nicht zur Fixierung genügt.

Weiterhin ist in der US 3,322,381 eine Halterung bekannt, bei der die Informationsanzeige in eine Klammer befestigt wird.
In der DE 202 02 774 U1 wird eine Vitrine beschrieben, die an einem Rohr in unterschiedlichen Höhen befestigt werden kann.
In der US 5,215,379 wird eine Tasche für Informationsmaterialien beschrieben, die mit Klettbändern an einem Pfosten befestigt werden kann.

In der US 2007/0299358 A1 wird ein Rollwagen beschrieben, an dessen Gestänge Ablageflächen lösbar eingehängt werden können.

In der WO 2010/107455 A1 wird eine einklemmbare Stange beschrieben, an der ebenfalls Ablageflächen angebracht werden können.

Der Erfindung liegt die Aufgabe zugrunde, eine Halterung für die Anzeige solcher Informationen direkt an einer Stange eines medizinischen Geräts, z.B, einer Infusionsstange, zur Verfügung zu stellen, die einfach, ohne Zuhilfenahme von Werkzeugen befestigt und auch wieder entfernt werden kann. Weiterhin soll diese Halterung einfach herzustellen, zu reinigen und zu desinfizieren sein, sowie aus möglichst wenigen Komponenten bestehen.

Nach der Lehre der Erfindung wird diese Aufgabe durch eine Halterung nach den Merkmalen des Anspruchs 1 und der Verwendung der Halterung gemäß Anspruch 15 gelöst. Vorteilhafte Ausgestaltungen sind Grundlage der Unteransprüche.

Die Halterung für die Anbringung einer Informationsanzeige, z.B. einer Gebrauchsanweisung, an einer Stange, z.B. der Infusionsstange, eines medizinischen Gerätes weist die Form eines weitgehend rechteckigen Rahmens auf. Sie besteht aus einer Basisplatte und einer Aufsteckplatte, an denen einstückig mindestens zwei Befestigungsmittel ausgebildet sind. Basis- und Aufsteckplatte werden über Steck- und Klemmvorrichtungen lösbar miteinander verbunden. Sie bilden dann eine kassettenartige Vorrichtung in Form eines Rahmens, der zur Aufnahme der Informationsanzeige oder Gebrauchsanweisung geeignet ist. Die Gebrauchsanweisung kann z.B. in Form eines Buches vorliegen. Die Basisplatte bildet die Rückwand des kassettenartigen Rahmens. Sie ist weitgehend rechteckig ausgebildet und weist vorzugsweise mittig einen weitgehend rechteckigen Ausschnitt aus. Dieser Ausschnitt an der Basisplatte ist nicht obligatorisch, da die Gebrauchsanweisung von der Rückseite nicht lesbar sein muss. Der Ausschnitt vermindert allerdings die Reibung beim Einschieben der Gebrauchsanweisung und bedingt auch einen geringeren Materialaufwand. Die Aufsteckplatte bildet die Vorderwand der Kassette. Sie entspricht in ihrer Größe der Basisplatte. Die Aufsteckplatte weist ebenfalls bevorzugt einen mittigen Ausschnitt auf, der als Sichtfenster dienen kann. Die Größe und Form der Basis- und Aufsteckplatte werden so dimensioniert, dass die Halterung stabil ist und vorzugsweise ein genügend großes Sichtfenster vorhanden ist. So kann die Länge die Außenkanten z.B. 20-25 cm X 15-20 cm betragen. Der Ausschnitt in der Mitte der Platten kann z.B. eine Größe von 14-19 cm X 9-14 cm aufweisen. Es sind aber auch andere Dimensionen geeignet, die dem Mittel, das zur Informationsanzeige verwendet werden soll (z.B. der Gebrauchsanweisung), angepasst sind.

Die weitgehend rechteckige Aufsteckplatte weist an drei Außenkanten Fortsätze auf, die vertikal zu der Hauptfläche der Aufsteckplatte ausgebildet sind und als Abstandshalter zur Basisplatte dienen. Diese Abstandshalter bedingen den kassettenartigen Aufbau der Halterung. Die Länge der Abstandshalter sollte der Dicke des Mittels zur Informationsanzeige angepasst sein. Sie kann z.B. zwischen 1,5-5 cm betragen.

An der Basisplatte einstückig ausgebildet sind die Mittel zur Befestigung der Halterung an einer Stange, z.B. der Infusionsstange. Die Basisplatte wird mit den Befestigungsmitteln als ein Spritzgussteil hergestellt. Dabei ist das Befestigungsmittel an der oberen Außenkante der Basisplatte als geschlossene, feste Schlinge ausgebildet. Mit dieser Schlinge wird die Halterung am oberen Ende der Stange, z.B. der Infusionsstange, an der die Informationsanzeige, z.B. die Gebrauchsanweisung, angebracht werden soll, eingehängt. Diese feste Schlinge verhindert eine Drehung der Halterung um die Längsachse der Stange. An der unteren Außenkante der Basisplatte einstückig ausgebildet, befindet sich das zweite Befestigungsmittel. Es ist z.B. ein Haken, der so dimensioniert ist, dass er mit der Stange in Form einer Schnappverbindung verklemmt werden kann. Alternativ dazu kann dieses Befestigungsmittel auch als Klammer aus zwei aufeinander zulaufender Fortsätzen ausgebildet sein, die in die Stange eingeklemmt werden kann. Diese Befestigungsmittel ermöglichen eine sehr einfache Montage der Halterung an der Stange und ebenso ein sehr einfache Entfernung von der Stange, z.B. der Infusionsstange, durch den Anwender. Es werden keine Werkzeuge benötigt, Anbringung und Entfernung sind mit einer Hand durchführbar.

In einer alternativen Ausführungsform ist die feste Schlinge, die zur Einhängung an der Stange dient, an der oberen Außenkante der Basisplatte ausgebildet, während das Befestigungsmittel zur Verklemmung mit der Stange, z.B. der Infusionsstange, an der Aufsteckplatte angeordnet ist.

In einer weiteren alternativen Ausführungsform sind beide Befestigungsmittel an der Aufsteckplatte ausgebildet. Diese Ausführungsform hat den Vorteil, dass die Basisplatte mit einem besonders einfachen Spritzgusswerkzeug herzustellen ist.

Zur Verbindung der Basisplatte mit der Aufsteckplatte werden Schnapp- bzw. Klemmverbindungen verwendet. Damit besteht die Halterung lediglich aus zwei Teilen, nämlich Basis- und Aufsteckplatte, die mittels Steck- und Klemmvorrichtungen lösbar miteinander verbunden sind. Dadurch ist die Halterung sehr gut zu reinigen und zu desinfizieren.

An drei Seiten weist die Basisplatte Mittel auf, die zur Aufnahme der Verbindungsmittel der Aufsteckplatte dienen.

An den Außenkanten der Basisplatte befinden sich Aussparungen in Form von Ausschnitten, in die die Klemmhaken der Aufsteckplatte eingreifen und auch einrasten können.

An der Aufsteckplatte sind an drei Außenkanten Abstandshalter in vertikaler Richtung zur Hauptfläche ausgebildet. Diese Abstandshalter sind mit Klemmhaken versehen, die geeignet sind, mit den Aussparungen der Basisplatte Schnappverbindungen einzugehen.

In einer alternativen Ausführungsform ist der Abstandshalter der unteren Außenkante mir Einführnasen versehen, die ohne Verklemmung in die Ausschnitte eingeführt werden. Beim Zusammenbau dieser Halterung werden die Einführnasen der Aufsteckplatte in die entsprechenden Ausschnitte der Basisplatte eingehängt. Aufsteckplatte und Basisplatte werden dann in einer Schwenkbewegung aufeinander zubewegt und mit den Klemmverbindungen aneinander fixiert.

In einer weiteren alternativen Ausführungsform sind die Klemmmittel an der oberen Außenkante mit Fortsätzen versehen, die an die Haken für die Aufhängung von Infusionslösungen anliegen und so eine Verdrehung um die Längsachse der Stange, z.B. der Infusionsstange, verhindern. Sie bilden zusätzlich zu der festen Schlinge einen Verdrehschutz für die Halterung.

An einer seitlichen Außenkante der Aufsteckplatte und der Basisplatte befinden sich keine Abstandshalter. Der aus Basisplatte und Aufsteckplatte bebildete Rahmen ist an dieser Seite offen. Hier kann die Gebrauchsanweisung in die Halterung eingeschoben werden.

In einer bevorzugten Ausführungsform ist an dieser seitlichen Außenkante der Aufsteckplatte und ebenso der entsprechenden Außenkante der Basisplatte eine Einführschräge vorgesehen. Diese Einführschräge erleichtert das Einschieben der Gebrauchsanweisung.

Die Halterung wird vorzugsweise aus Kunststoff, wie z.B. PET oder ABS, gefertigt, der die nötige Elastizität aufweist, um die beschriebenen Klemmverbindungen herzustellen.

In einer alternativen Ausführungsform ist die Halterung aus Blech gefertigt.

Weitere Einzelheiten und Vorteile der Erfindung werden anhand der in den Zeichnungen dargestellten Ausführungsbeispiele näher beschrieben. Sie zeigen:
Figur 1: Perspektivische Vorderansicht einer ersten Ausführungsform der erfindungsgemäßen Halterung mit Gebrauchsanweisung an einer Infusionsstange
Figur 2: Perspektivische Rückansicht der Halterung nach Figur 1 mit Gebrauchsanweisung an der Infusionsstange
Figur 3: Perspektivische Ansicht der Halterung nach Figur 1 in demontiertem Zustand
Figur 4: Perspektivische Ansicht einer zweiten Ausführungsform der erfindungsgemäßen Aufsteckplatte
Figur 5: Perspektivische Ansicht der Ausführungsform der Halterung nach Figur 4
Figur 6: Perspektivische Ansicht dritten Ausführungsform der Aufsteckplatte der erfindungsgemäßen Halterung.

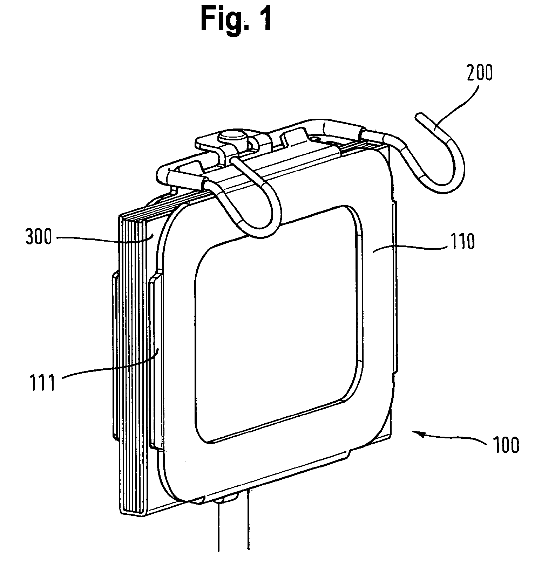

Figur 1 zeigt in perspektivischer Ansicht von schräg vorne eine erster Ausführungsform der erfindungsgemäßen Halterung 100 montiert an einer Infusionsstange 200. Die Gebrauchsanweisung 300 ist in die Halterung 100 eingeschoben und wird in dem Rahmen aus Basisplatte 150 und Aufsteckplatte 110 dem Bediener präsentiert. Die Aufsteckplatte 110 weist dabei an der linken, seitlichen Außenkante eine Einführschräge 111 auf, die das Einschieben der Gebrauchsanweisung 300 erleichtert.

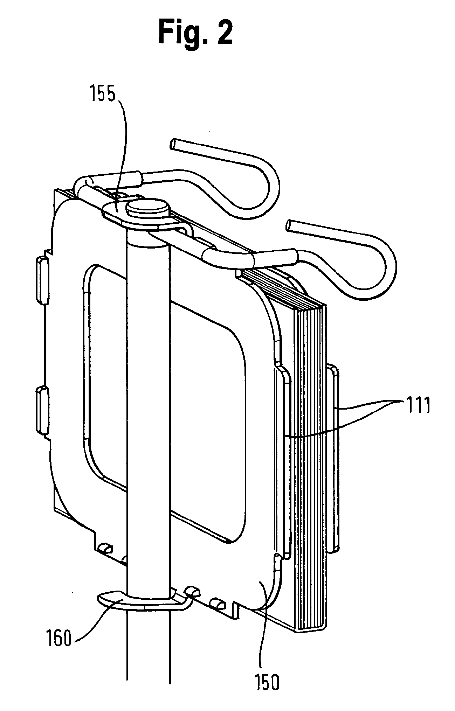

In Figur 2 wird die gleiche Ausführungsform in einer perspektivischen Ansicht von schräg hinten gezeigt. Hier ist die Basisplatte 150 gezeigt mit geschlossenen Klemmverbindungen mit der Aufsteckplatte 110. An der Basisplatte einstückig ausgebildet sind die beiden Befestigungsmittel 155 und 160. Sie bestehen aus einer festen Schlinge 155 an der oberen Außenkante der Basisplatte. Diese wird in das obere Ende der Infusionsstange eingehängt.

Das zweite Befestigungsmittel 160 an der unteren Außenkante der Basisplatte 150 besteht aus einem Haken 160, der mit der Infusionsstange verklemmt wird. Wie die Aufsteckplatte 110 weist auch die Basisplatte 150 an einer seitlichen Außenkante eine Einführschräge 111 auf, die das Einschieben der Gebrauchsanweisung erleichtert.

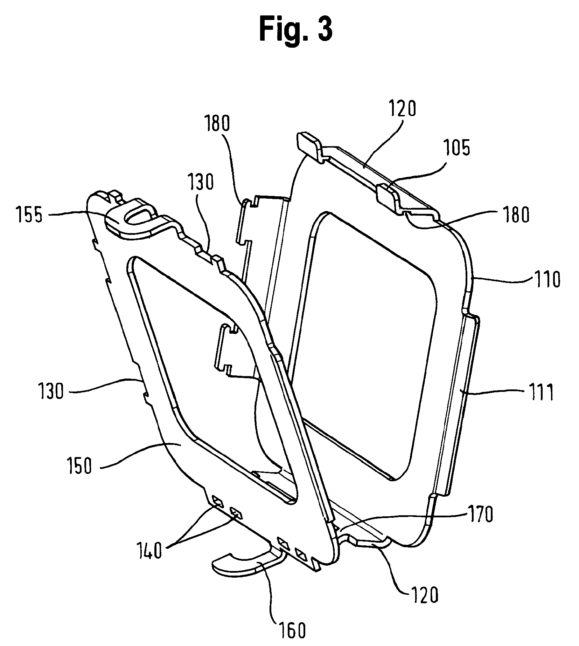

Figur 3 zeigt eine perspektivische Ansicht der Halterung 100 in demontiertem Zustand, bei der die Klemmverbindungen zwischen der Basisplatte 150 und der Aufsteckplatte 110 voneinander gelöst sind. An der Basisplatte 150 befinden sich die Befestigungsmittel 155 und 160. Desweiteren sind an der oberen und der linken Außenkante die Ausschnitte 130 für die Klemmhaken 180 und im unteren Rahmenteil die Ausschnitte 140 für die Einführnasen 170 dargestellt. An der vierten Außenkante ist die Einführschräge 111 vorgesehen. An der Aufsteckplatte 110 sind an drei Außenkanten Fortsätze als Abstandshalter 120 vorgesehen. An der unteren Außenkante sind an dem Abstandshalter 120 Einführnasen 170 ausgebildet. Die beiden anderen Abstandshalter 120 weisen Klemmhaken 180 auf, die zur Herstellung der Klemmverbindung der Aufsteckplatte mit der Basisplatte dienen. Die Abstandshalter an der oberen Außenkante weisen zusätzliche Fortsätze auf, die als Verdrehschutz 105 dienen. Die Einführnasen 170 werden in die entsprechenden Ausschnitte 140 ohne Verklemmung eingesetzt. Basisplatte 150 und Aufsteckplatte 110 werden mit einer Schwenkbewegung über die Klemmverbindungen miteinander verbunden.

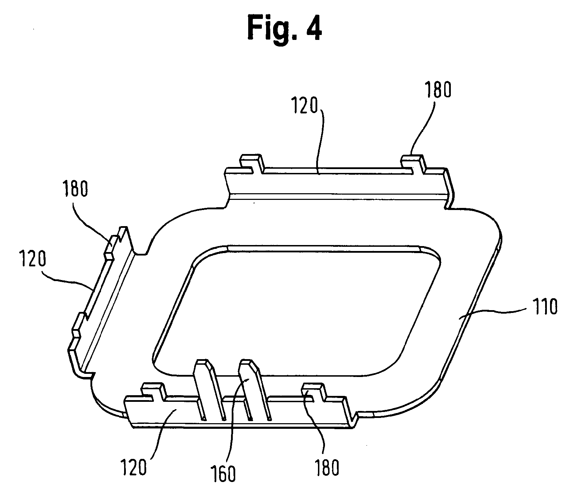

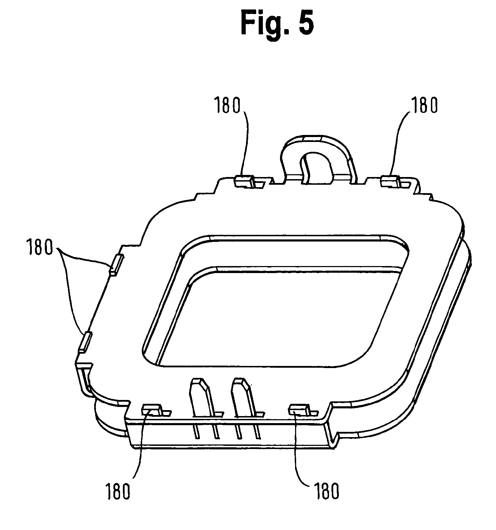

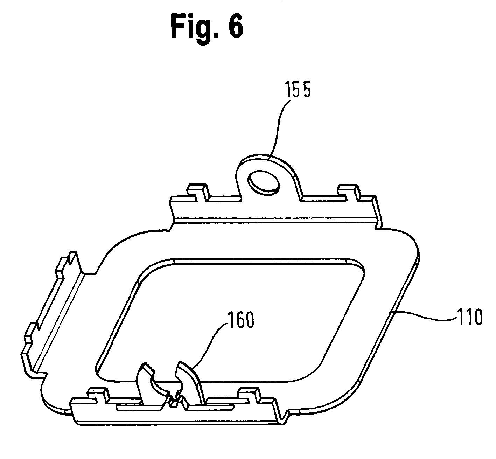

Figur 4 zeigt eine zweite Ausführungsform der erfindungsgemäßen Aufsteckplatte 110 bei der das Befestigungsmittel 160 als Klammer an derselben ausgebildet ist. Bei dieser Ausführungsform befinden sich zusätzliche Ausschnitte an der Basisplatte, durch die das Befestigungsmittel 160 bei der Montage der Halterung 100 aus Basisplatte 150 und Aufsteckplatte 110 durchgeführt wird. Figur 5 zeigt die Halterung 100 mit der Aufsteckplatte 110 aus Figur 4. Figur 6 zeigt eine dritte Ausführungsform der erfindungsgemäßen Aufsteckplatte 110, bei der Befestigungsmittel 155 und 160 an der Aufsteckplatte ausgebildet sind.

## Patentansprüche

1. Halterung (100) für die Anbringung einer Informationsanzeige (300) an einer Stange (200) an einem medizinischen Gerät in Form eines rechteckigen Rahmens, der aus einer Basisplatte (150) und einer Aufsteckplatte (110) besteht, wobei die Basisplatte (150) und die Aufsteckplatte (110) mittels Steck- und Klemmvorrichtungen (130, 140, 170, 180) lösbar miteinander verbunden sind, und wobei die Halterung mindestens zwei Mittel zur Befestigung (155, 160) an der Stange umfasst, welche einstückig an der Basisplatte(150) und/oder der Aufsteckplatte (110) ausgebildet sind **gekennzeichnet dadurch, dass** an drei Außenkanten der Aufsteckplatte (110) Fortsätze (120) vertikal zur Hauptfläche der Aufsteckplatte (110) ausgebildet sind, die als Abstandshalter (120) zur Basisplatte dienen.

2. Halterung (100) nach Anspruch 1 **gekennzeichnet dadurch, dass** das erste Mittel zur Befestigung als einhängbare, feste Schlinge (155) ausgebildet ist.

3. Halterung (100) nach Anspruch 1 oder 2 **gekennzeichnet dadurch, dass** das zweite Mittel zur Befestigung als Haken (160) ausgebildet ist, der mit der Stange verklemmt werden kann.

4. Halterung (100) nach Anspruch 1 oder 2 **gekennzeichnet dadurch, dass** das zweite Mittel zur Befestigung als Klammer ausgebildet ist, die mit der Stange (160) verklemmt werden kann.

5. Halterung (100) nach einem der vorhergehenden Ansprüche **gekennzeichnet dadurch, dass** die Basisplatte (150) an drei Außenkanten Aussparungen (130) aufweist, die zur Aufnahme von Einführnasen (170) und Klemmhaken (180) der Aufsteckplatte (160) dienen.

6. Halterung (100) nach einem der vorhergehenden Ansprüche **gekennzeichnet dadurch, dass** die Außenkante einer Seite der Basisplatte (150) mit einer Einführschräge (111) versehen ist.

7. Halterung (100) nach einem der vorangehenden Ansprüche **gekennzeichnet dadurch, dass** mindestens zwei an Seiten befindliche Abstandshalter (120) mit Klemmhaken (180) versehen sind.

8. Halterung (100) nach einem der vorangehenden Ansprüche **gekennzeichnet dadurch, dass** die Abstandshalter (120) einer Seite der Aufsteckplatte (110) mit Einführnasen (170) versehen ist.

9. Halterung (100) nach einem der vorhergehenden Ansprüche **gekennzeichnet dadurch, dass** die Außenkante einer Seite der Aufsteckplatte (110) mit einer Einführschräge (111) versehen ist.

10. Halterung (100) nach Anspruch 1 **gekennzeichnet dadurch, dass** sich an der Aufsteckplatte (110) weiterhin Mittel (105) zur Verhinderung der Rotation der Halterung um die Stange befinden.

11. Halterung (100) nach einem der vorhergehenden Ansprüche **gekennzeichnet dadurch, dass** die Anbringung an einer Infusionsstange (200) erfolgt.

12. Halterung (100) nach einem der vorhergehenden Ansprüche **gekennzeichnet dadurch, dass** es sich bei der Informationsanzeige um eine Gebrauchsanweisung handelt.

13. Verwendung einer Halterung (100) nach Anspruch 1 zur Bereitstellung von Informationen an einem medizinischen Gerät.

14. Verwendung nach Anspruch 13, wobei es sich bei dem medizinischen Gerät um eine Dialysemaschine handelt.

## Claims

1. A holder (100) for mounting an information display (300) on a rod (200) on a medical device in the form of a rectangular frame, consisting of a base plate (150) and an mounted plate (110), wherein the base plate (150) and the mounted plate (110) are detachably connected to one another by means of plug and clamping devices (130, 140, 170, 180), and wherein the holder comprises at least two means for fastening (155, 160) on the rod, designed in one piece on the base plate (150) or on the mounted plate (110), **characterized in that** protrusions (120) are designed vertically to the main surface of the mounted plate (110) on three outside edges of the mounted plate (110), serving as spacers (120) from the base plate.

2. The holder (100) according to Claim 1, **characterized in that** the first means are designed for fastening as a fixed loop (155) that can be suspended.

3. The holder (100) according to Claim 1 or 2, **characterized in that** the second means for fastening are designed as a hook (160), which can be clamped onto the rod.

4. The holder (100) according to Claim 1 or 2, **characterized in that** the second means for fastening are designed as a clamp, which can be clamped onto the rod (160).

5. The holder (100) according to any one of the preceding claims, **characterized in that** the base plate (150) has recesses (130) on three outside edges, serving to receive insertion noses (170) and clamping hooks (180) on the mounted plate (160).

6. The holder (100) according to any one of the preceding claims, **characterized in that** the outside edge of one side of the base plate (150) is provided with an insertion chamfer (111).

7. The holder (100) according to any one of the preceding claims, **characterized in that** at least two spacers (120) situated on sides are provided with clamping hooks (180).

8. The holder (100) according to any one of the preceding claims, **characterized in that** these spacers (120) are provided with insertion noses (170) on one side of the mounted plate (110).

9. The holder (100) according to any one of the preceding claims, **characterized in that** the outside edge on one side of the mounted plate (110) is provided with an insertion chamfer (111).

10. The holder (100) according to Claim 1, **characterized in that** means (105) for preventing rotation of the holder about the rod are also provided on the mounted plate (110).

11. The holder (100) according to any one of the preceding claims, **characterized in that** the holder is mounted on an infusion pole (200).

12. The holder (100) according to any one of the preceding claims, **characterized in that** the information display comprises instructions for use.

13. A use of a holder (100) according to Claim 1 for supplying information to a medical device.

14. The use according to Claim 13, wherein the medical device is a dialysis machine.

## Revendications

1. Fixation (100) pour appliquer un affichage d'informations (300) sur une barre (200) sur un appareil médical, ayant la forme d'un cadre rectangulaire composé d'une plaque de base (150) et d'une plaque de pose (110), sachant que la plaque de base (150) et la plaque de pose (110) sont reliées entre elles de façon séparable au moyen de dispositifs d'encliquetage et de serrage (130, 140, 170, 180), et sachant que la fixation comprend au moins deux moyens de fixation (155, 160) sur la barre qui sont formés d'un seul tenant sur la plaque de base (150) ou la plaque de pose (110), **caractérisée en ce que** des prolongements (120) sont formés sur trois arêtes extérieures de la plaque de pose (110), verticalement à la face principale de la plaque de pose (110), lesquels servent d'écarteurs (120) par rapport à la plaque de base.

2. Fixation (100) selon la revendication 1, **caractérisée en ce que** le premier moyen de fixation est formé en tant que boucle (155) fixe pouvant être accrochée.

3. Fixation (100) selon la revendication 1 ou 2, **caractérisée en ce que** le second moyen de fixation est formé en tant que crochet (160) qui peut être coincé avec la barre.

4. Fixation (100) selon la revendication 1 ou 2, **caractérisée en ce que** le second moyen de fixation est formé en tant qu'attache qui peut être coincée avec la barre (160).

5. Fixation (100) selon l'une des revendications précédentes, **caractérisée en ce que** la plaque de base (150) présente des évidements (130) sur trois arêtes extérieures qui servent à recevoir des ergots d'introduction (170) et des crochets de serrage (180) de la plaque de pose (160).

6. Fixation (100) selon l'une des revendications précédentes, **caractérisée en ce que** l'arête extérieure d'un côté de la plaque de base (150) est dotée d'un biseau d'insertion (111).

7. Fixation (100) selon l'une des revendications précédentes, **caractérisée en ce qu'**au moins deux écarteurs (120) se trouvant sur les côtés sont dotés de crochets de serrage (180).

8. Fixation (100) selon l'une des revendications précédentes, **caractérisée en ce que** les écarteurs (120) d'un côté de la plaque de pose (110) sont dotés d'ergots d'introduction (170).

9. Fixation (100) selon l'une des revendications précédentes, **caractérisée en ce que** l'arête extérieure d'un côté de la plaque de pose (110) est dotée d'un biseau d'insertion (111).

10. Fixation (100) selon la revendication 1, **caractérisée en ce que** des moyens (105) pour empêcher la rotation de la fixation sur la barre se trouvent en outre sur la plaque de pose (110).

11. Fixation (100) selon l'une des revendications précédentes, **caractérisée en ce que** l'application est effectuée sur une barre de perfusion (200).

12. Fixation (100) selon l'une des revendications précédentes, **caractérisée en ce que** l'affichage d'informations est une notice d'utilisation.

13. Emploi d'une fixation (100) selon la revendication 1 pour mettre à disposition des informations sur un appareil médical.

14. Emploi selon la revendication 13, dans lequel l'appareil médical est une machine de dialyse.
